# EUROPEAN PATENT SPECIFICATION

(11) **EP 4 252 764 B1**
(45) Date of publication and mention of the grant of the patent: **17.12.2025**
(21) Application number: 22166365.1
(22) Date of filing: 01.04.2022
(51) Int. Cl.: C12R 1/01, C12N 1/20, A61K 35/00, A23L 33/135

(54) **PARABACTEROIDES DISTASONIS STRAIN ISOLATED FROM HUMAN FECES AND APPLICATIONS THEREOF**
PARABACTEROIDES DISTASONIS-STAMM ISOLIERT AUS MENSCHLICHEN KOT UND ANWENDUNGEN DAVON
SOUCHE DE PARABACTEROIDES DISTASONIS ISOLÉE À PARTIR DE MATIÈRES FÉCALES HUMAINES ET SES APPLICATIONS

(43) Date of publication of application: 04.10.2023
(73) Proprietor: Greentech, 63360 Saint-Beauzire (FR)
(72) Inventor: GERVASON, Sandie, 63430 Pont-du-château (FR); FILAIRE, Edith, 63730 Mirefleurs (FR); BERTHON, Jean-Yves, 63540 Romagnat (FR); CARVALHO, Frédéric, 63000 Clermont-Ferrand (FR)
(74) Representative: Touroude, Magali Linda

(56) References cited:
- EP-A1- 3 967 314
- WO-A1-2016/075294
- WO-A1-2019/075344
- WEST CHRISTINE ET AL: "Animal models of visceral pain and the role of the microbiome", NEUROBIOLOGY OF PAIN, vol. 10, 1 August 2021 (2021-08-01), pages 100064, XP55941950, ISSN: 2452-073X, Retrieved from the Internet <URL:https://www.ncbi.nlm.nih.gov/pmc/articles/PMC8190503/pdf/main.pdf> DOI: 10.1016/j.ynpai.2021.100064
- G. SISSON ET AL: "Randomised clinical trial: a liquid multi-strain probiotic vs. placebo in the irritable bowel syndrome - a 12 week double-blind study", ALIMENTARY PHARMACOLOGY & THERAPEUTICS., vol. 40, no. 1, 1 July 2014 (2014-07-01), GB, pages 51 - 62, XP055333765, ISSN: 0269-2813, DOI: 10.1111/apt.12787

## Description

The present invention concerns a *Parabacteroides distasonis* strain deposited under accession number CNCM I-5828. This strain can be useful in multiple applications, in particular incorporated in a nutritional supplement or in a composition for use as a medicament, especially in the prevention or treatment of visceral pain, particularly observed in Inflammatory Bowel Disease including Crohn's disease and Ulcerative Colitis, or in Irritable Bowel Syndrome.

### Technical Field

This invention is in the field of bacterial strains isolated from human, especially human feces, and compositions including this strain. In preferred embodiments, the invention is in the field of nutritional supplement composition and composition for use as a medicament including this strain. This composition for use as a medicament is especially for preventing or treating visceral pain.

### Background Art

The microbiome comprises several types of microorganisms (bacteria, archaea, viruses, parasites and fungi), their genomes, and their surrounding environment, including the gastrointestinal tract, oral mucosa, urogenital and respiratory systems, and the skin surface [1]. Bacteria are the group of microorganisms that compose the microbiome which is most studied [2]. It is estimated that their number in the entire human body is of the same order as the number of human cells [3]. Many bacteria reside in the intestines [4]. Due to their huge number and diversity, they can significantly affect normal physiology and modify the host's susceptibility to diseases [5]. Indeed, changes in microbiota composition have been documented in Inflammatory Bowel Disease (IBD) and in Irritable Bowel Syndrome (IBS).

Crohn's disease (CD) and ulcerative colitis (UC) are part of IBD and are characterized by inflammation of epithelial barrier of intestinal tract. [6]. The pattern of dysbiosis mostly associated with IBD patients is a decrease in commensal bacteria diversity, and a relative increase of bacterial species belonging to *Enterobacteriaceae* [7]. While data are heterogeneous, the gut microbiota appears to play an important role in IBD pathogenesis. A recently published study conducted on 132 IBD patients provided the most comprehensive description to date of host and microbial activities in IBD, demonstrating that the gut microbiome, the molecular functional profile and the host immune factors are key component in IBD [8]. Beside to identify microbial, transcriptomic and metabolomic profiles, recent studies highlighted metabolites signature in patients suffered from IBD [9]. Similarly, another recently published study showed a disruption in the taxa of the *Lachnospiraceae* and *Ruminococcaceae* families in IBD patients compared to controls, and demonstrated that disturbance in the distinct networks of taxa associations is involved in CD and UC disease development [8].

In recognition of the potential positive effect that several bacterial strains may have on the animal gut, various strains have been proposed as new therapeutic strategies in various diseases. Several strains, including mostly *Lactobacillus* and *Bifidobacterium* strains, have also been proposed for treatment of inflammatory and autoimmune diseases that are not directly linked to the gastrointestinal tract. However, the relationship between different diseases and different bacterial strains, and precise effects of bacterial strains on the gut and at a systemic level and on any types of diseases are poorly characterized.

IBS or spastic colon is a disorder characterized by abdominal pain and changes in bowel habits, which are not associated with any abnormalities seen on routine clinical diagnostic. It is common and makes up 20-50% of visits to gastroenterologists. Chronic pain within the digestive tract remains a major unmet medical problem. It accompanies common digestive diseases, is poorly understood and is difficult to treat.

Lower abdominal pain, and bloating associated with alteration of bowel habits and abdominal discomfort relieved with defecation are the most frequent symptoms. It must be understood for the good comprehension of the present invention that IBS is a syndrome and that under this expression are collected several symptoms observed on patients suffering from the gastro-intestinal tract. Thus, therapeutic solutions aim to treat or to improve several symptoms associated to IBS.

Visceral pain is a common symptom observed in Inflammatory Bowel Disease (IBD: Crohn's disease, ulcerative colitis). Pain is an important manifestation of inflammation, as inflammatory cytokines and mediators sensitize primary afferent neurons. It should thus not be surprising that pain is one of the presenting symptoms in about 50%-70% of patients experiencing the initial onset or exacerbations of IBD [10]. The current treatment options are mostly focusing on the reduction of inflammation for IBD or the intestinal motility disturbances for IBS. Only a few therapies aim directly at diminishing abdominal pain [11]. Today, only symptomatic treatments are proposed and alternatives solutions are currently explored to relieve visceral and abdominal pain in patients suffering from IBD or IBS. Among, them, adapted diet, fecal microbiota transplantation or use of "biotics" products (including probiotics, prebiotics, synbiotics, metabiotics or parabiotics) are explored strategies to restore microbiota homeostasis and to limit inflammation.

At the intestinal level, the stimuli of pain can be varied, both mechanical (intestinal peristalsis, distension, stretching), or chemical (neurotransmitters or inflammation mediators for example) and can come from various origins: both intestinal cells, immunity cells or the intestinal microbiota. There are many receptors involved in pain signaling. Nociceptive stimuli, whether mechanical, chemical, or thermal, are picked up by nociceptors called primary afferent neurons and carry information to the posterior horn of the spinal cord. In pain perception process, transmission of sensorial information from the intestine to the brain is ensured by extrinsic nervous system whose cell bodies localized in dorsal root ganglia (DRG) playing a crucial function and relaying nociceptive information to the brain. Spinal afferences play a crucial role in the transmission of information about physiological disorders, causing bloating, discomfort or visceral pain. As a true sentinel system, extrinsic innervation ensures communication between the intestine and the brain, transmitting digestive and physiological information to the central nervous system where it will be treated. This is the pathway that nerve impulses use to get to the central nervous system. Pain perception involves complex cellular and molecular mechanisms in several stages. In the first step, intestinal information is encoded and relayed to the posterior horn of the spinal cord. This is the transduction step. During transduction, the nociceptive stimulus depolarizes the free termination of nociceptors, thereby activating the opening of voltage-dependent sodium channels and causing the generation of an action potential (PA). This nerve impulse induces the massive entry of positively charged ions at the central terminus in the posterior horn of the spinal cord and is transmitted to a secondary neuron up to the thalamus where it will synapse with a third neuron. This is the transmission phase in the brain. The latter then directs the nociceptive information to different regions of the somatosensory cortex, capable of distinguishing sensory information from affective or emotional information.

Animal models of colitis have been studied to develop alternative treatment options for patients with visceral pain, by measuring colonic hypersensitivity. In addition to exhibiting responses to inflammation that are similar to those seen in humans, rodent models have allowed the identification of unique characteristics of populations of sensory neurons that innervate the gut. Interestingly, there are common features of sensory neurons innervating different organs including colon, stomach, and pancreas. All these structures are densely innervated by sensory fibers that express calcitonin gene-related peptide (CGRP). This peptide, a potent vasodilator, is released by peripheral sensory nerve terminals in response to noxious and non-noxious stimulation and can have effects on vasculature and other structures. This property highlights one of the underappreciated aspects of visceral (and most somatic) sensory neurons; ie, in addition to detecting internal (visceral) or external (environmental) stimuli, sensory neurons release a number of compounds including CGRP and substance P (SP) that can have peripheral motor and/or autocrine/paracrine effects on other cells or sensory neurons. Under normal conditions, these compounds contribute to homeostatic regulation, but in disease states, including those with an inflammatory component, they may exacerbate symptoms ("neurogenic inflammation"). Most TRPV1-expressing neurons release SP and/or CGRP and it is thought that the release of these compounds may drive inflammatory responses.

IBD and IBS are characterized, among other things, by the development of visceral hypersensitivity. Several mediators may be at the origin such as serotonin, histamine, bradykinin, substance P, ATP, prostaglandins as well as all mediators of inflammation such as cytokines and chemokines. These molecules interact with neuronal receptors involved in the signaling of visceral pain. Activation of these receptors can lead to an anti-nociceptive or, on the contrary, pro-nociceptive effect depending on whether they are activators or inhibitors and participate to pain modulation. Several receptors such as serotoninergic, histaminergic, bradykinin receptors, or receptors belonging to the TRP (Transient receptor potential) family such as TRPV1, TRPV4, Transient receptor potential ankyrin 1 (TRPA1), but also purinergic or tachykinin receptors involvement in modulation of visceral painful process have been studied and quickly became therapeutic targets. One of them, TRPV1, may play a central role in setting the overall sensitivity of colonic afferences. In fact, colonic hypersensitivity developed in response to inflammatory mediators in wild-type mice is absent in TRPV1-deficient mice. In addition, TRPV1 is overexpressed and correlated with pain severity in IBD [13] and in IBS [14].

Moreover, pharmacologic inhibition of the TRPV1 blocks the development of hyperalgesia during colitis [15]. Whereas TRPV1 has received considerable attention recently, no single channel is likely to be responsible for colonic hypersensitivity. Deletion of a member of acid sensitive ion channels (ASIC3) or another member of the TRP (transient receptor potential) family (TRPV4) blunt or completely block the development of hyperalgesia in response to colonic inflammation [15]. TRPA1 has also been reported to contribute to visceral pain-like behavior in dextran sulfate sodium (DSS)-evoked colitis. Pain process involves complex molecular pathways and various neuronal receptors that can, together, contribute to generation of pain. This is the case of G-coupled protein receptor (GPCR) whose activation can sensitize nociceptors belonging to TRP family. This is the reason why GPCR has become therapeutic target for the treatment of chronic pain in the digestive system [16].

The article West C. et al. (West, C., & Neufeld, K. A. M. (2021). Animal models of visceral pain and the role of the microbiome. Neurobiology of Pain, 10, 100064) teaches about animal models used in the development of visceral pain therapies (microbiota-related therapies). This document list different mice models for visceral pain, including chemical-induced models, post-infections models and stress-induced models. This article present colorectal distension assay as one commonly used method that replicates human experience of visceral pain, and a list of probiotics successfully used to minimize symptoms of visceral pain in animal models.

EP3967314 relates to bacterial strains of the species *Parabacteroides distasonis* for use in the treatment and/or prevention of a gastrointestinal disease, or of a disorder associated with gastrointestinal disease.

The article Sisson G. et al. (Sisson, G., Ayis, S., Sherwood, R. A., & Bjarnason, I. (2014). Randomised clinical trial: a liquid multi-strain probiotic vs. placebo in the irritable bowel syndrome-a 12 week double-blind study. Alimentary pharmacology & therapeutics, 40(1), 51-62.) discloses the use of a liquid probiotic (different *Lactobacillus* strains) in human patients suffering from visceral pain caused by Irritable Bowel Syndrome.

WO2019075344 relates to the use of fecal microbiota transplantation (FMT) for the treatment of severe bloating and abdominal pain, e.g. during defecation, in a human patient. This document discloses that FMT results in changes in the patient's microbiota profile towards the donor species (Bacteroides spp.).

WO2016075294 relates to the use of *Faecalibacterium prausnitzii* CNCM 1-4573 to treat visceral pain of Neonatal Maternal Separation (NMS) colonic hypersensitivity mouse model.

*Parabacteroides distasonis* is a bacterium belonging to *Porphyromonadaceae* family and to *Parabacteroides* genus. Initially classified in the *Bacteroides* genus based on its phenotypic and morphological characteristics, this bacterial species was recently reclassified and belongs to the *Tannerellaceae* including *Parabacteroides* and *Tannerella* genera. Indeed, beyond its phenotypic characteristics, this species proves to be closer phylogenetically to the species *Tannerella fosythensis.* The genus *Parabacteroides* currently counts 15 species : *Parabacteroides acidifaciens, Parabacteroides bouchesdurhonensis, Parabacteroides chartae, Parabacteroides chinchillae, Parabacteroides chongii, Parabacteroides distasonis, Parabacteroides faecis, Parabacteroides goldsteinii, Parabacteroides gordonii, Parabacteroides johnsonii, Parabacteroides massiliensis, Parabacteroides merdae, Parabacteroides pacaensis, Parabacteroides provencensis, Parabacteroides timonensis. Parabacteroides distasonis* represents the reference species and the strain P. *distasonis* ATCC 8503 (DSM20701) is the reference strain to the genus *Parabacteroides* to have been reclassified [17].

*Parabacteroides distasonis* is the type of strain for the genus *Parabacteroides,* a group of gram-negative anaerobic bacteria that commonly colonize the gastrointestinal tract of numerous species. Anti-inflammatory properties of P. *distasonis* have been demonstrated [18]. In addition to exert anti-inflammatory properties, some P. *distasonis* strains have demonstrated beneficial effect on strengthening of epithelial barrier.

For the above reasons, there is a requirement for new ways of treating visceral pain, in particular induced by IBS or IBD. Moreover, there is a constant need to identify new bacterial strains which can be useful in many technical domains, and especially in nutritional supplements and as medicaments.

Surprisingly, the Applicant identified from human feces a new strain of the genus *Parabacteroides,* from the species *Parabacteroides distasonis,* which demonstrates an unexpected and strong efficacy to prevent or treat visceral pain.

### Disclosure of the invention

Therefore, in a first aspect, the invention concerns a *Parabacteroides distasonis* strain deposited under accession number CNCM I-5828.

The *Parabacteroides distasonis* strain according to the invention has a 16s rRNA gene sequence as shown in SEQ ID NO:1.

The *Parabacteroides distasonis* strain according to the invention has been isolated from healthy human feces.

The bacterial strain according to the invention has been named "P. *distasonis* F1-2". This name will be used especially in the examples to describe the strain according to the invention and deposited under accession number CNCM I-5828, and having a 16s rRNA gene sequence as shown in SEQ ID NO:1.

The bacterial strain used in the invention has been characterized in particular by sequencing 16s rRNA gene. The 16s rRNA gene sequence (SEQ ID NO:1) of this bacterial strain is at least 99.61% identical to the 16s RNA sequence of the species *Parabacteroides distasonis (Parabacteroides distasonis* ATCC 8503). 16s rRNA gene sequencing is commonly used as a tool to identify bacteria at the species level and assist with differentiating between closely related bacterial species. The sequence of the complete genome of *Parabacteroides distasonis* ATCC 8503 is known in the art, and can be found on the following link: https://www.ebi.ac.uk/ena/browser/api/fasta/CP000140.1?lineLimit=1000

The bacterial strain of the invention has been isolated from healthy male human.

In a second aspect, the invention concerns a composition comprising a *Parabacteroides distasonis* strain deposited under accession number CNCM I-5828.

In an embodiment, said composition can further comprises any type of other compounds which could be suitable for the purpose of the composition.

In a third aspect, the invention concerns a nutritional supplement composition comprising a bacterial strain according to the invention.

By "nutritional supplement", "Food product", "Food supplement", "nutraceutical composition" or "Dietary supplement" is identically intended according to the invention a product that may provide nutritional benefit. This supplement can be taken alone or be formulated with other compounds in order to make the composition more attractive to consume by being more similar to a common food item. This supplement can be a contributing factor to prevent or diminished any superficial visceral pain problem which does not need a therapeutic treatment.

In an embodiment, the nutritional supplement composition further comprises at least one acceptable nutritional ingredient, preferably chosen among: probiotics, prebiotics, symbiotics, parabiotics, metabiotics, vitamins, minerals, herbs, amino acids and enzymes.

Probiotics intends according to the invention to live microorganisms that, when administered in adequate amounts, confer a health benefit on the host.

More preferably, said probiotics are chosen among bacteria belonging *to Bifidobacterium, Lactobacillus, Lactococcus, Streptococcus* genus or next generation probiotics such as bacterial strains identified as *Faecalibacterium prausnitzii, Akkermansia muciniphila, Bacteroides fragilis, Bacteroides thetaiotaomicron, Clostridium butyricum, Roseburia hominis, Roseburia intestinalis, Hafnia alvei, or Christensenella minuta.* It comprises also *Saccharomyces* fungi strains.

Prebiotics intends according to the invention to selectively fermented ingredients that results in specific changes in the composition and/or activity of the gastrointestinal microbiota, thus conferring benefit(s) upon host health.

More preferably, said prebiotics are chosen among Galactooligosaccharides, fructooligosaccharides or polysaccharides.

Symbiotics intends according to the invention to a mixture of probiotics and prebiotics that beneficially affects the host by improving the survival and activity of beneficial microorganisms in the gut.

More preferably, said synbiotics are chosen among bacteria belonging *to Bifidobacterium, Lactobacillus, Lactococcus, Streptococcus* genus or next generation probiotics such as bacterial strains identified as *Faecalibacterium prausnitzii, Akkermansia muciniphila, Bacteroides fragilis, Bacteroides thetaiotaomicron, Clostridium butyricum, Roseburia hominis, Roseburia intestinalis, Hafnia alvei, or Christensenella minuta.* It comprises also *Saccharomyces* fungi strains. All these bacteria or fungi should be associated with prebiotics such as galacooligosaccharides, fructooligosaccharides or polysaccharides in symbiotic formulation.

Parabiotics, paraprobiotics or inactivated probiotics intends according to the invention to non-viable microbial cells (either intact or broken) or crude cell extracts which when administered (either orally or topically) in adequate amounts, confer a benefit on the human or animal consumer.

More preferably, said parabiotics are chosen among: Inactivated microbial cells belonging to *Lactobacillus, Lactococcus, Streptococcus* or *Bifidobacterium* genus or based on *Faecalibacterium prausnitzii, Akkermansia muciniphila, Bacteroides fragilis, Bacteroides thetaiotaomicron, Clostridium butyricum, Roseburia hominis, Roseburia intestinalis, Hafnia alvei, or Christensenella minuta bacterial species or Saccharomyces* fungi strains.

Metabiotics or "postbiotics" are bioactive molecules produced by probiotics which provides physiological benefits to the host, by regulating the interactions between the host and the gut microbiome, for example through epigenetic regulation and cell communication. They can be found in any product which has been fermented by living bacteria.

Preferably, said metabiotics are chosen among products which have been fermented with living bacteria.

In an embodiment, the nutritional supplement composition according to the invention comprises at least 10⁹, preferably at least 10¹⁰, colony forming units/mL of the *Parabacteroides distasonis* strain deposited under accession number CNCM I-5828.

In an embodiment, the administration of this nutritional supplement is by oral take.

In an embodiment, the taken of the nutritional supplement is made by one or several successive doses.

In an embodiment, the successive doses are separated by at least 12 hours, preferably at least one day, more preferably at least two days, even more preferably at least 3 days, even more preferably at least 4 days, even more preferably at least 5 days, even more preferably at least 6 days.

In an even more preferred embodiment, said successive doses are separated by at least 1 week.

In an embodiment, the oral take of the nutritional supplement is made by gelatin capsules, capsules, tablets, powders, granules, oral solutions or suspensions.

In an embodiment, the oral take of the nutritional supplement is made through food products, beverage, a food additive, or a milk product, containing said nutritional supplement.

In an embodiment, the nutritional supplement composition according to the invention only comprises living bacteria.

In an embodiment, the nutritional supplement composition according to the invention only comprises killed bacteria and/or fractions of bacteria obtained by thermal, chemical or mechanical inactivation or UV inactivation that could be fractionated or purified.

According to the invention, a "killed bacteria" is a bacteria strain which is inactivated and which is no longer able to replicate.

The killed bacteria could be obtain according to the invention by heating treatment at 121°C during 15 to 30 minutes.

In an embodiment, the nutritional supplement composition according to the invention comprises living bacteria and/or killed bacteria and/or fractions of bacteria.

In a fourth aspect, the invention concerns a composition comprising the bacterial strain according to the invention, for use as a medicament.

In an embodiment, the composition is for use in treating and/or preventing visceral pain.

In a preferred embodiment, said visceral pain is induced by Inflammatory Bowel Disease including Crohn's disease and Ulcerative Colitis, or by Irritable Bowel Syndrome.

"Visceral pain" according to the invention includes chronic abdominal pain, thoracic pain, pelvic pain, bloating associated with alteration of bowel habits, abdominal discomfort relieved with defecation, chronic pain within the digestive tract. According to the invention, this term includes pain related to the stomach, pancreas, liver, gallbladder, colon, and small and large intestine.

According to the invention, the term "preventing" indicates a reduction of the risk to develop the said pathologies.

According to the invention, the term "treating" means the alleviation of the symptoms associated with said specific disorders or conditions and/or elimination of said symptoms.

As shown in the examples of this application, this bacterial strain exhibits unusual anti-nociceptive properties by reducing visceral hypersensitivity in inflammatory and non-inflammatory mouse model that was not observed with the reference bacterial strain belonging to the same species. In addition, *P. distasonis* F1-2 strain exerts unusual neuromodulatory properties that contributes to decrease pain processing and reduces the severity of visceral pain, including antalgic effect. Moreover, this composition reduces pain observed in IBS. Finally, this strain of *Parabacteroides distasonis* has neuroinhibitory properties on nociceptors expressing TRPV1 channel and G-coupled protein receptors.

In an embodiment the composition for use according to the invention comprises at least 10⁹, preferably at least to 10¹⁰ colony forming units/mL of the *Parabacteroides distasonis* strain deposited under accession number CNCM I-5828.

In an embodiment the composition for use according to the invention only comprises living bacteria.

In an embodiment the composition for use according to the invention only comprises killed bacteria and/or fractions of bacteria.

In an embodiment the composition for use according to the invention comprises living bacteria and/or killed bacteria and/or fractions of bacteria.

In an embodiment, the composition for use according to the invention further comprises at least one other acceptable active pharmaceutical ingredient, and/or at least one acceptable pharmaceutical excipient and/or at least one acceptable pharmaceutical carrier.

As acceptable active pharmaceutical ingredient is intended according to the invention every compound, molecule or else which can be useful to add into the composition to reach the purpose of the composition according to the invention. This definition includes especially anti-inflammatory molecules such as for example naproxen, aspirin or acetaminophen, as well as opioids such as codeine or morphine, or painkiller.

As acceptable pharmaceutical excipient is intended according to the invention any substances other than the active ones, that have been appropriately evaluated for safety and are intentionally included in a formulation. These excipients enable the medicament to be effectively administered to the patient, and to support the right delivery into the body.

As acceptable pharmaceutical carrier is intended according to the invention any substrate which serves to improve the selectivity, effectiveness, and/or safety of the administration of the composition according to the invention.

In an embodiment, the composition for use according to the invention is formulated to be suitable for oral, or in intestine administration.

In a preferred embodiment, the composition for use according to the invention is administered orally, through gelatin capsules, capsules, tablets, powders, granules, oral solutions or suspensions.

In an embodiment, the administration is made by one or several successive doses.

In an embodiment, the successive doses are separated by at least 12 hours, preferably at least one day, more preferably at least two days, even more preferably at least 3 days, even more preferably at least 4 days, even more preferably at least 5 days, even more preferably at least 6 days.

Preferably, said successive doses are separated by at least 1 week.

In an embodiment, the subject is a mammal, including non-human mammal, and is, in particular, a human being.

In a preferred embodiment, the subject is a human adult.

In another aspect, the invention concerns a cell of the *Parabacteroides distasonis* strain deposited under accession number CNCM I-5828.

In another aspect, the invention concerns a cell of the *Parabacteroides distasonis* strain deposited under accession number CNCM I-5828, for use as a medicament.

In an embodiment, the cell according to the invention is for use in treating and/or preventing visceral pain.

More preferably, said visceral pain is induced by Inflammatory Bowel Disease including Crohn's disease and Ulcerative Colitis, or by Irritable Bowel Syndrome.

In another aspect, the invention concerns a nutritional supplement composition comprising a cell of the *Parabacteroides distasonis* strain deposited under accession number CNCM I-5828.

In another aspect, the invention concerns a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In a preferred embodiment, said strain has a 16s rRNA gene sequence that is at least 85.00%, more preferably at least 90.00%, even more preferably at least 95.00%, identical to the 16s RNA sequence of a bacteria strain of the species *Parabacteroides distasonis* chosen among *Parabacteroides distasonis* ATCC 8503 or SEQ ID NO:1.

In a preferred embodiment, said strain has a 16s rRNA gene sequence that is at least 96.00%, more preferably at least 97.00%, even more preferably at least 98.00%, identical to the 16s RNA sequence of a bacteria strain of the species *Parabacteroides distasonis* chosen among *Parabacteroides distasonis* ATCC 8503 or SEQ ID NO:1.

In a preferred embodiment, said strain has a 16s rRNA gene sequence that is at least 99.00%, more preferably at least 99.50%, identical to the 16s RNA sequence of a bacteria strain of the species *Parabacteroides distasonis* chosen among *Parabacteroides distasonis* ATCC 8503 or SEQ ID NO:1.

In a preferred embodiment, said strain has a 16s rRNA gene sequence that is at least 99.61%, identical to the 16s RNA sequence of the bacteria strain *Parabacteroides distasonis* ATCC 8503 or SEQ ID NO:1.

In a preferred embodiment, said strain has a 16s rRNA gene sequence as described in SEQ. ID NO:1.

In another aspect, the invention concerns a composition comprising a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In an embodiment, the composition according to the invention comprises at least 10⁹, preferably at least 10¹⁰, colony forming units/mL of a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In an embodiment, said composition comprises a strain which is at least 85.00%, preferably at least 90.00%, more preferably at least 95.00%, more preferably at least 96.00%, more preferably at least 97.00%, more preferably at least 98.00%, more preferably at least 99.00%, more preferably at least 99.50%, more preferably at least 99.61%, identical to the 16s RNA sequence of a bacteria strain of the species *Parabacteroides distasonis* chosen among *Parabacteroides distasonis* ATCC 8503 or SEQ ID NO:1.

In another aspect, the invention concerns a nutritional supplement composition comprising a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In an embodiment, the nutritional supplement composition according to the invention comprises at least 10⁹, preferably at least 10¹⁰, colony forming units/mL of a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In an embodiment, said nutritional supplement composition comprises a strain which is at least 85.00%, preferably at least 90.00%, more preferably at least 95.00%, more preferably at least 96.00%, more preferably at least 97.00%, more preferably at least 98.00%, more preferably at least 99.00%, more preferably at least 99.50%, more preferably at least 99.61%, identical to the 16s RNA sequence of a bacteria strain of the species *Parabacteroides distasonis* chosen among *Parabacteroides distasonis* ATCC 8503 or SEQ ID NO:1.

In another aspect, the invention concerns a composition comprising a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1, for use as a medicament.

In an embodiment, the composition for use according to the invention comprises at least 10⁹, preferably at least 10¹⁰, colony forming units/mL of a strain of *Parabacteroides distasonis* which has a 16s rRNA gene sequence that is at least 80.00% identical to the 16s RNA sequence of a bacteria strain of the species *Parabacteroides distasonis* chosen among *Parabacteroides distasonis* ATCC 8503 or SEQ ID NO:1.

In an embodiment, said composition for use comprises a strain which is at least 85.00%, preferably at least 90.00%, more preferably at least 95.00%, more preferably at least 96.00%, more preferably at least 97.00%, more preferably at least 98.00%, more preferably at least 99.00%, more preferably at least 99.50%, more preferably at least 99.61%, identical to the 16s RNA sequence of a bacteria strain of the species *Parabacteroides distasonis* chosen among *Parabacteroides distasonis* ATCC 8503 or SEQ ID NO:1.

In an embodiment, said composition is for use in treating and/or preventing visceral pain. In an embodiment, said visceral pain is induced by Inflammatory Bowel Disease including Crohn's disease and Ulcerative Colitis, or by Irritable Bowel Syndrome.

References to a percentage sequence identity between two nucleotide sequences means that, when aligned, that percentage of nucleotides are the same in comparing the two sequences.

### Brief Description of Drawings

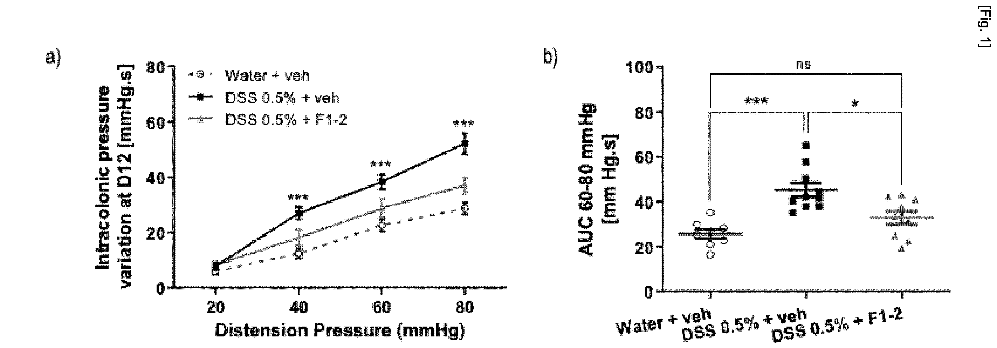

[Fig.1]: *P. distasonis F1-2* reverses colonic hypersensitivity in dextran sulfate sodium 0.5% colitis induced mouse model. (a) Visceromotor response to colorectal distension in control (vehicle) mice (n = 8), DSS treated mice (n = 10) and P. *distasonis* F1-2 treated mice (n = 9). (b) The area under curve (AUC) was calculated between 60 and 80 mmHg for the different groups. P. *distasonis* F1-2 strain administration significantly alleviated colonic hypersensitivity induced by DSS treatment in mice. Mean +/- SEM. Statistical analysis: (a) Two-way ANOVA followed by Dunnett's multiple comparisons test. DSS 0.5% + veh vs Water + veh treated mice: ***p value = 0.0002 (at 80mmHg); ***p value = 0.0006 (at 60mmHg); ***p value = 0.0002 (at 40mmHg). DSS 0.5% + F1-2 vs Water + veh treated mice: no significant (40mmHg p value = 0.1945; 60mmHg p value = 0.2234; 80mmHg p value = 0.0589. (b) One-way ANOVA followed by Tukey's multiple comparisons test. Water + veh vs DSS 0.5% + veh treated mice: ***p value = 0.0002; Water + veh vs DSS 0.5% + F1-2: no significant (p value = 0.2015); DSS 0.5% + veh vs DSS 0.5% + F1-2 treated mice: *p value = 0.0108.

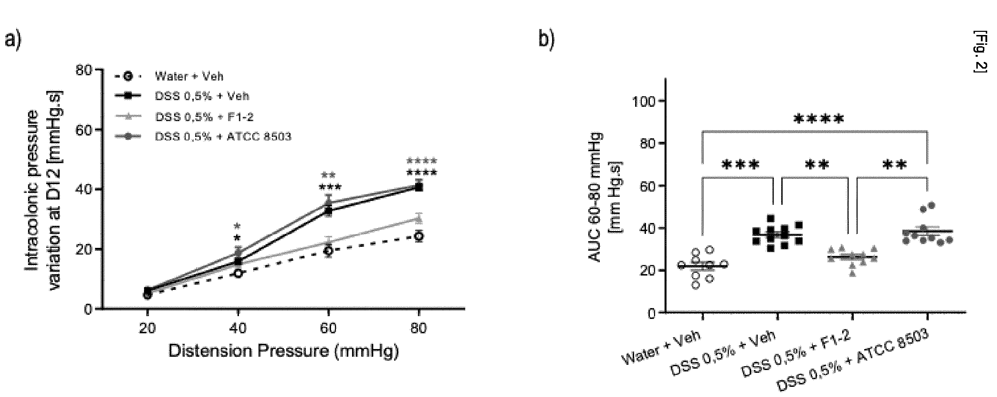

[Fig.2]: Administration of *Parabacteroides distasonis* F1-2 but not P. *distasonis* ATCC 8503 prevents colonic hypersensitivity in DSS-induced colitis. (a) Visceromotor response to colorectal distension in control (water + vehicle) mice (n = 9), DSS 0,5% + vehicle treated mice (n = 11), DSS 0,5% + P. *distasonis* F1-2 (n = 10) or DSS 0,5% + P. *distasonis* ATCC 8503 (n = 10) treated mice was assessed at D12. (b) The area under curve (AUC) was calculated between 60 and 80mmHg for the different groups. Mean +/- SEM. Statistical analysis: (a) Two-way ANOVA followed by Tukey's multiple comparisons test. Water + veh vs DSS 0,5% + veh treated mice: ****p value<0,0001 (at 80mmHg); ***p value = 0,001 (at 60mmHg); *p value = 0,0243 (at 40mmHg). Water + veh vs DSS 0,5% + ATCC 8503 treated mice: ****p value<0,0001 (at 80mmHg); **p value = 0,0012 (at 60mmHg); *p value = 0,0343 (at 40mmHg). Water + veh vs DSS 0,5% + F1-2 treated mice: ns (at 80, 60 and 40mmHg).

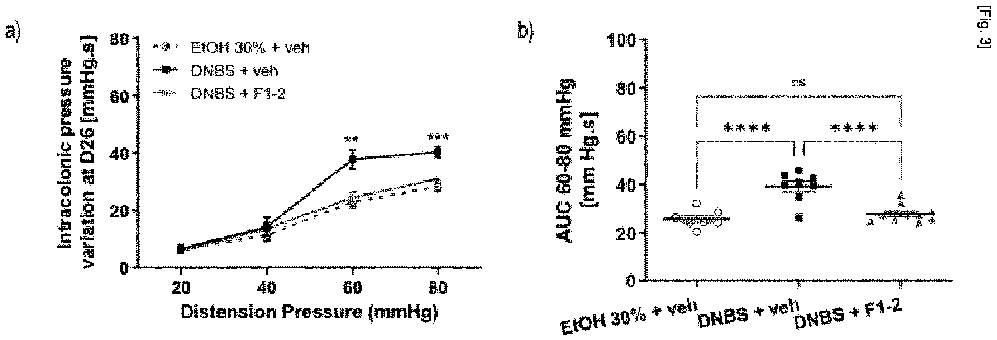

[Fig.3]: *Parabacteroides distasonis* F1-2 alleviates colonic hypersensitivity in post-inflammatory DNBS colitis induced mouse model. (a) Visceromotor response to colorectal distension in EtOH + vehicle mice (n = 7), DNBS + vehicle treated mice (n = 8) and DNBS + P. *distasonis* F1-2 treated mice (n = 10). (b) Area under curve (AUC) was calculated between 60 and 80 mmHg for the different groups. *P. distasonis* F1-2 administration significantly alleviated colonic hypersensitivity induced by DNBS treatment. Mean +/- SEM. Statistical analysis: (a) Two-way ANOVA followed by Dunnett's multiple comparisons test. EtOH 30% + veh vs DNBS + veh treated mice: ***p value = 0.0003 (at 80mmHg); **p value = 0.0038 (at 60mmHg). DNBS + F1-2 vs EtOH 30% + veh treated mice: no significant 80mmHg p value = 0.2504; 60mmHg p value = 0.7811. (b) One-way ANOVA followed by Tukey's multiple comparisons test. EtOH 30% + veh vs DNBS + veh treated mice: ****p value<0.0001. EtOH 30% + veh vs DNBS + F1-2 treated mice: ns (p value = 6270). DNBS + veh vs DNBS + F1-2 treated mice: ****p value<0.0001.

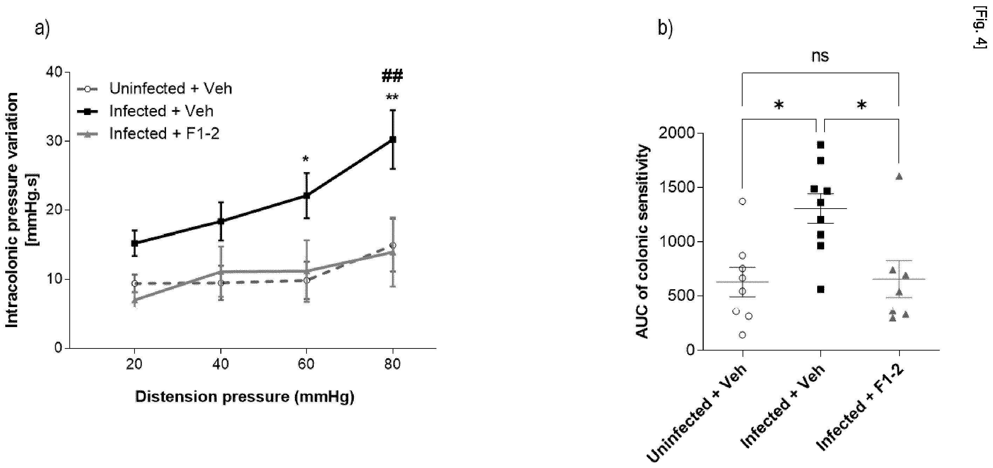

[Fig.4]: *P. distasonis* F1-2 alleviates colonic hypersensitivity in post-infectious IBS mouse model. (a) Visceromotor response to colorectal distension in uninfected mice (n=8), Infected + vehicle mice (n=9) and P. *distasonis* F1-2 infected treated mice (n=7). (b) Area under curve between 60 and 80 mmHg for the different groups. Colonic hypersensitivity developed by infected mice in post-infectious phase was reversed by P. *distasonis* F1-2 treatment. Mean +/- SEM. Statistical analysis: (a) Two-way ANOVA followed by Tukey's multiple comparisons test. Uninfected + veh vs Infected + veh treated mice: **p value = 0.0028 (at 80mmHg); *p value = 0.0202 (at 60mmHg). Infected + veh vs Infected + F1-2 treated mice: ##p value = 0.002. (b) Kruskal-Wallis test. Uninfected + veh vs Infected + veh treated mice: *p value = 0.0267. Infected + veh vs Infected + F1-2 treated mice: *p value = 0.0355. Uninfected + veh vs Infected + F1-2: no significant (p value > 0.99).

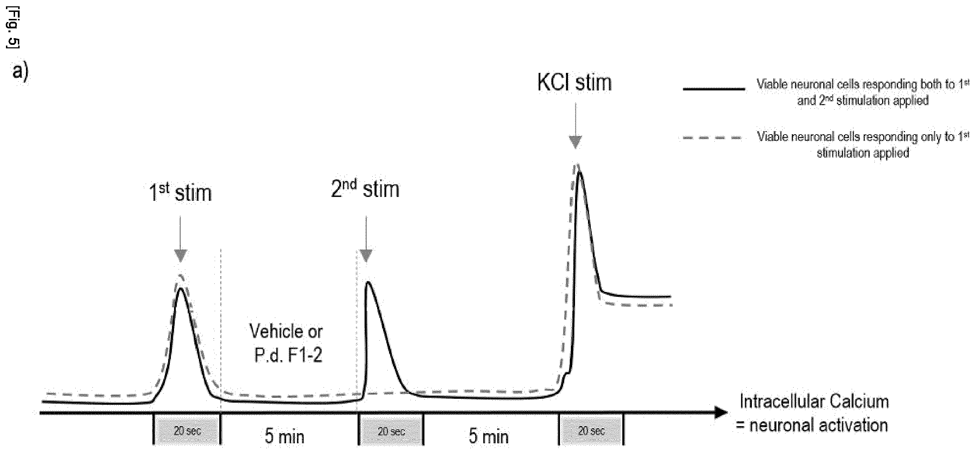

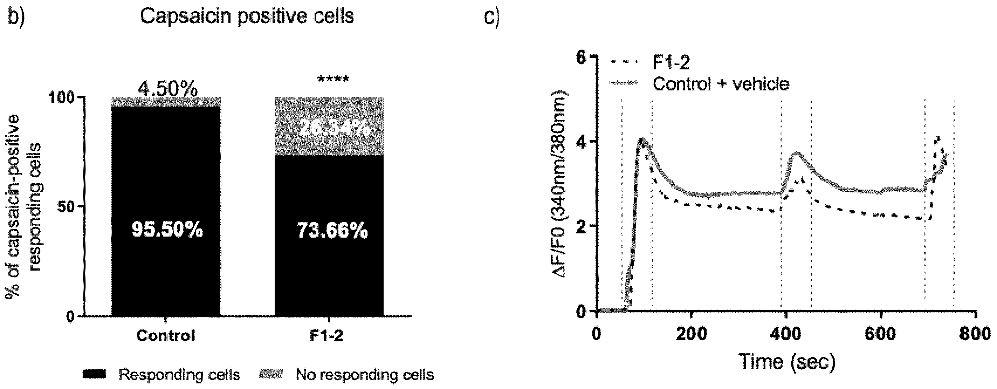

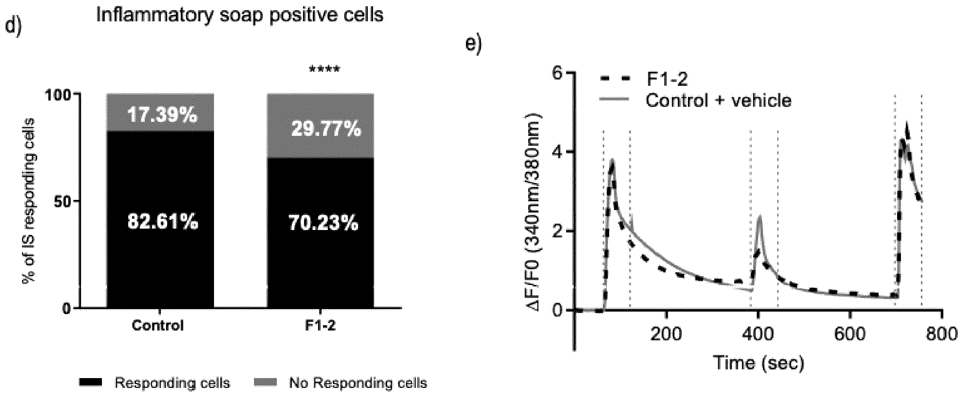

[Fig.5]: *P. distasonis* F1-2 decreases nociceptors activation level. (a) Experimental protocol. Schematic representation of activation signal observed by ratiometric calcium imaging with 2 sub populations. First one corresponds to neuronal cells responding both to the first and the second stimulation (in black). The second corresponds to neuronal cells that responding only to the first stimulation (in grey). Neuronal cells were stimulated a first time with capsaicin (1µM) or IS before being incubated with Tyrode or P. *distasonis* F1-2. Neuronal cells were stimulated a second time with capsaicin or IS mixed with P. *distasonis* F1-2 before being lastly stimulated with KCI (50mM). IS was composed by bradykinin, serotonin, histamine, PGE2 and KCI. (b) % of sub population of neuronal cells activated by capsaicin. (c) Global kinetic signal activation measured as normalized fluorescence 340nm/380nm for capsaicin positive responding cells in each group. (d) % of sub population of neuronal cells activated by IS. (e) Global kinetic signal activation measured as normalized fluorescence 340nm/380nm for IS positive responding cells in each group. Statistical analysis: Chi square test ****p value<0,0001. *GPCR: G-protein coupled receptor. IS: Inflammatory soap.*

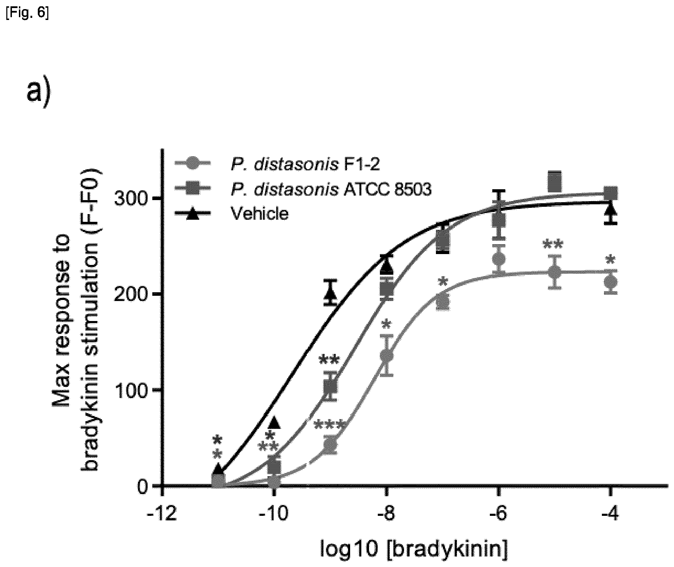

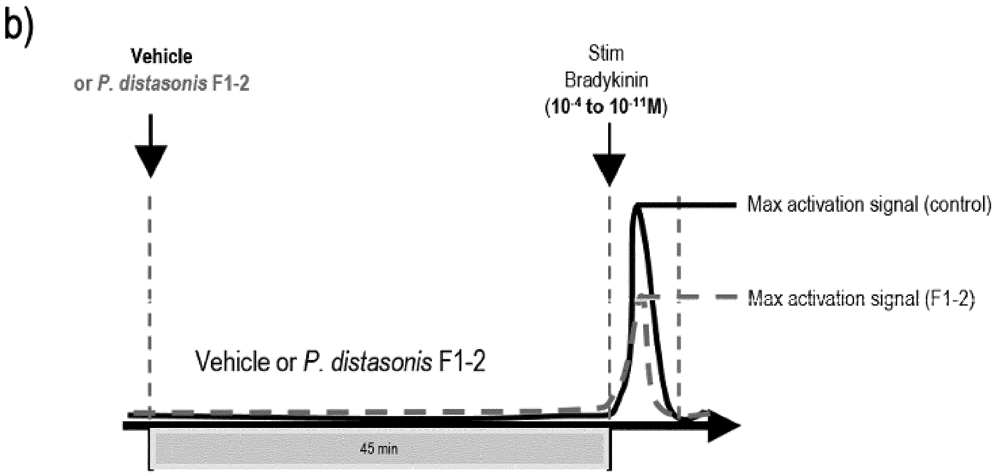

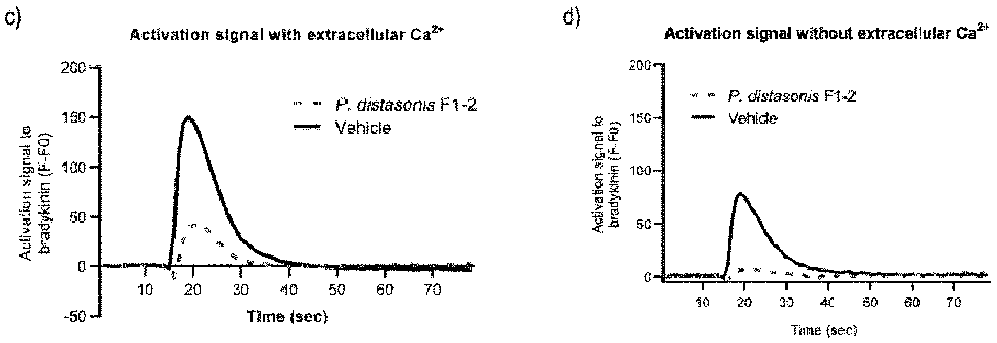

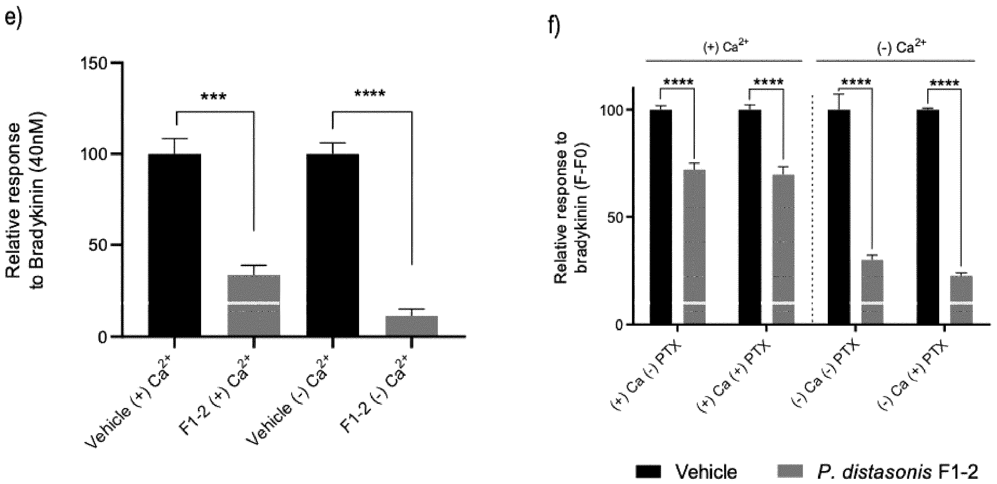

[Fig.6]: *P. distasonis* F1-2 neuroinhibitory potential could involve G-protein coupled receptor. (a) Dose-response curve of bradykinin on ND7/23 expressing bradykinin receptor incubated with or without P. *distasonis* F1-2 or P. *distasonis* ATCC 8503 strain. (b) Experimental protocol. Schematic representation of Ca²⁺ activation signal of ND7/23 neuronal cells loaded with calcium probe (FLIPR Calcium 6 QF) stimulated with bradykinin (40nM) after 45 min incubation with P. *distasonis* F1-2. (c) Activation signal of ND7/23 neuronal cells stimulated with bradykinin after 45 min incubation with P. *distasonis* F1-2 with extracellular Ca²⁺ or (d) in deprivation of extracellular Ca²⁺. (e) Relative response to control. Bar graphs represent maximum peaks values on normalized F-F0 relative to control. (f) Relative response to bradykinin stimulation of ND7/23 overnight pre-treated or not with pertussis toxin. Statistical analysis: (a) Mean +/- SEM. Two-way ANOVA followed by Dunnett's multiple comparisons test. *P. distasonis* F1-2 versus vehicle: *p value=0,0191 (at 10⁻¹¹M); **p value=0,0016 (at 10⁻¹⁰M); ***p value=0,0002 (at 10⁻⁹M); *p value=0,0205 (at 10⁻⁸M); *p value=0,0222 (at 10⁻⁷M); **p value=0,0091 (at 10⁻⁵M); *p value=0,0165 (at 10⁻⁴M). *P. distasonis* ATCC 8503 versus vehicle: *p value=0,0157 (at 10⁻¹¹M); *p value=0,0266 (at 10⁻¹⁰M); **p value=0,0042 (at 10⁻⁹M). Each point represents an average of 4 independent readings. (e) One-way ANOVA followed by Tukey's multiple comparisons test. Vehicle (+) Ca²⁺vs F1-2 (+) Ca²⁺: ***p value = 0,0003. Vehicle (-) Ca²⁺ vs F1-2 (-) Ca²⁺: ****p value<0,0001. Each bar graph represents an average of 3 independent readings. (f) Mean +/-SEM. Two-way ANOVA followed by Sidak's multiple comparisons test. ****p value<0,0001. Each bar graph represents an average of 6 independent readings.

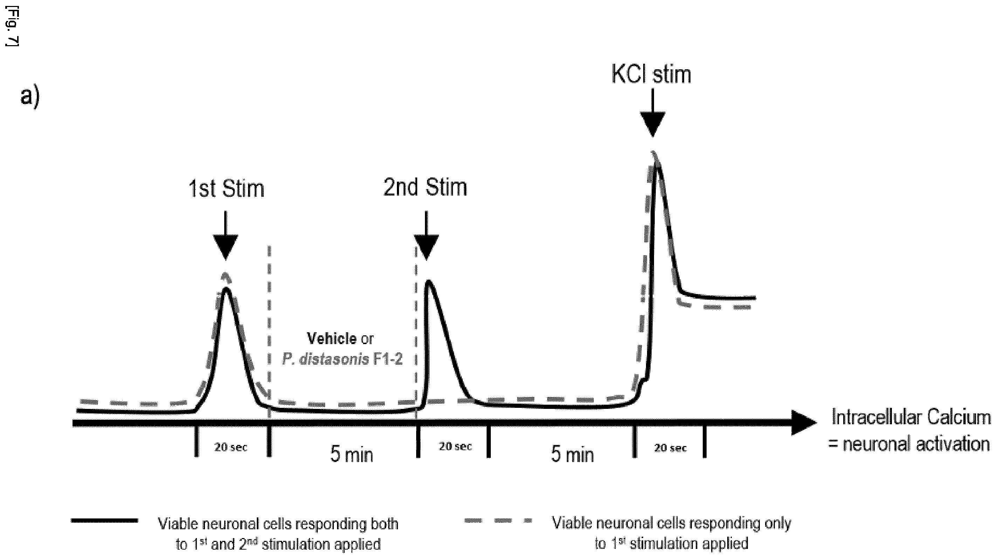

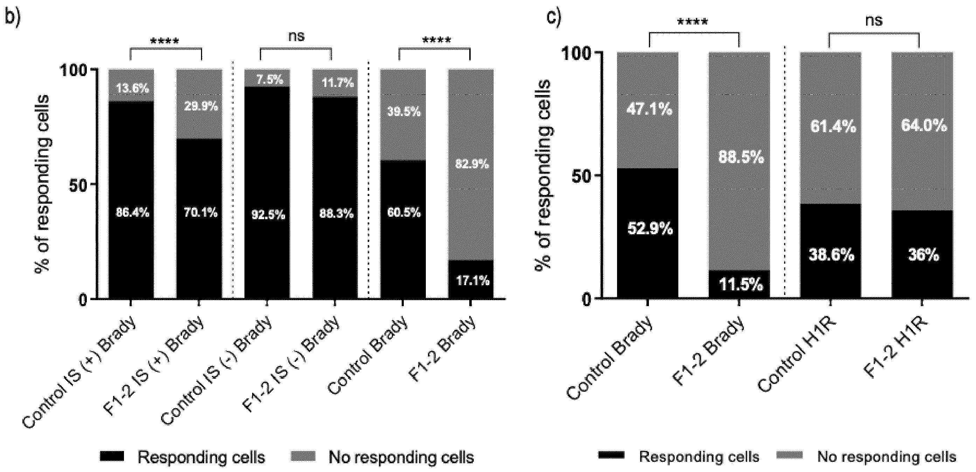

[Fig.7]: *P. distasonis* F1-2 neuroinhibitory potential could involve bradykinin receptor. (a) Experimental protocol. Schematic representation of activation signal observed by ratiometric calcium imaging with 2 sub populations. First one corresponds to neuronal cells responding both to the first and the second stimulation (in black). The second corresponds to neuronal cells that responding only to the first stimulation (in grey). Ca²⁺ responses were measured on neuronal cells from DRG mice as normalized fluorescence 340nm/380nm ratios. (b) % of sub population of neuronal cells activated by IS (+) bradykinin, IS (-) bradykinin or bradykinin. (c) % of sub population of neuronal cells activated by bradykinin or by histamin receptor 1 specific agonist (H1R) (2-pyridylethylamine). *F1-2: P. distasonis F1-2; Stim: stimulation; IS: Inflammatory soap; Brady: bradykinin; H1R: histamine receptor 1 specific agonist. Statistical analysis: Chi square test. ****p value<0,0001.*

### Examples

In the following examples, colonic hypersensitivity in animal models is measured to evaluate the efficacy of the strain according to the invention on chronic visceral pain in human.

### EXAMPLE 1: Parabacteroides distasonis F1-2 strain has antalgic effect in dextran sulfate sodium acute colitis mouse model (inflammatory visceral pain mouse model):

### PARABACTEROIDES DISTASONIS CULTURING METHODS

The strain *Parabacteroides distasonis* (isolate F1-2) has been cultivated in complex anaerobic medium containing Neutralized soya peptone (10g/L, OXOID), Yeast extract (15g/L, OXOID), KH₂PO₄ (0.9g/L), K₂HPO₄ (0.9g/L), NaCl (0.9g/L), sodium acetate (2.7g/L), MgSO₄,7H2O (0.09g/L), L-cysteine hydrochloride (1g/L), D-glucose -20g/L), NaHCO₃ (4g/L), Maltose (0.5g/L), Cellobiose (0.5g/L), adjusted at pH 6.9. For inoculum preparation, 1mL of P. *distasonis* F1-2 frozen stock culture was sub-cultured in a 50mL flask containing 25mL of anaerobic medium (previously described) and incubated 24h at 37°C in strict anaerobic conditions. A second sub-cultured was necessary and consists to transferred 24h first sub-cultured (OD600nm of 3-4) in 225 mL of anaerobic medium, incubated for 20h at 37°C in strict anaerobic conditions. Cultivation with growing cells was carried out in 5L bioreactor by inoculated 5% (v/v) of the second sub-cultured (OD600nm = 3) in the fresh anaerobic medium. Anaerobic conditions were maintained by continuous injection of a gas mixture (N₂ (85%), H₂ (5%) and CO₂ (10%)) allowing to maintain low Redox potential (-400mV) and homogenization was ensured by 2 Rushton blades with an agitation rate of 150 rpm. Bioreactor culture was conducted during 24h with a controlled temperature of 37°C and reached OD600nm of 6. Biomass of P. *distasonis* F1-2 was harvested by centrifuging whole culture 30 minutes at 5000g at 4°C in anaerobic condition to maintain cell viability and conserved in PBS + glycerol (20% (v/v)) solution at -80°C. Dry matter of biomass was adjusted to 19% with PBS + glycerol (20%) to standardized bacterial preparation. Bacterial titer was estimated between 1,2 x 10¹⁰ CFU/mL to 1,5 x 10¹⁰ CFU/mL according to the batch production by count on brucella blood agar medium (from anaerobe system).

### ANIMALS

57 Male C57/Blk6 mice (6-9 weeks old) were obtained from Janvier Labs (53 Le Genest-Saint-Isle. France).

### ANIMAL TREATMENTS

Acute colitis was induced by 0.5% (w/v) Dextran Sulfate Sodium (DSS) (mol wt = 36,000-50,000 Da) dissolved in drinking water for 12 days ad libitum. During 12 days, 0.2 mL of 1.52 x 10¹⁰ CFU/mL of *P*. *distasonis* F1-2 strain were daily administered by gavage. Animal weight loss was controlled during DSS administration and feces were harvested each day of the experiment and frozen at -80°C for further analysis. The study groups are as follows: control (water + vehicle group (PBS + glycerol (20%)), DSS + vehicle (PBS + glycerol (20%)) and DSS + F1-2 group. Colonic hypersensitivity was measured at the end of the experiment. In a second experiment, P. *distasonis* F1-2 strain antalgic effect was compared to P. *distasonis* ATCC 8503 reference strain following the same procedure.

### MEASUREMENT COLONIC HYPERSENSITIVITY BY COLORECTAL DISTENSION

Mice colonic sensitivity induced by DSS treatment was evaluated by quantifying visceromotor reaction in response to colorectal distensions (CRD). Mice were habituated 30 min prior to experiments, to reduce motion artefacts due to restraint stress. A polyethylene balloon with connecting catheter was inserted in the distal colon, 1 cm from the base of the balloon to the anus of mice, during light isoflurane anesthesia. The catheter was fixed to the tail with tape. The balloon was connected to pressure transducer to control intraballoon pressure during CRD procedure. Barostat was used to manage air infiltration and balloon pressure control. The CRD procedure allows assessing visceral pain-related responses and consisted of repeated phasic distensions of 20, 40, 60 and 80mmHg, with a pulse duration of 20 seconds and spaced from each other by 4 minutes.

### RESULTS - CONCLUSIONS

To evaluate anti-nociceptive effect of the strain P. *distasonis* F1-2 on colonic hypersensitivity, a model of colitis induced by 0.5% of Dextran sulfate sodium was used. *P. distasonis* F1-2 was administered by gavage each day throughout the duration of DSS treatment, so 12 consecutive days. While DSS 0.5% treatment increased significantly colonic hypersensitivity (***p value = 0.0002 (at 80mmHg)) (Figure 1a) (***p value = 0.0002) (Figure 1b), *P. distasonis* F1-2 oral administration significantly decreased colonic hypersensitivity compared to control. In fact, there is no significant difference between Water + veh and DSS 0.5% + F1-2 mice group (Figure 1a and Figure 1b) traducing a partial reversion of the treatment-induced painful phenotype.

In a second experiment, anti-nociceptive effect of P. *distasonis* F1-2 strain was compared to *P. distasonis* ATCC 8503 reference strain in the same visceral pain mouse model, according to the same procedure. While P. *distasonis* F1-2 strain exerted significant anti-nociceptive effect in mouse treated with dextran sulfate sodium (Water + veh vs DSS 0.5% + F1-2: no significant) (Figure 2a), P. *distasonis* ATCC 8503 reference strain had no beneficial effect on colonic hypersensitivity (Water + veh vs DSS 0.5% + ATCC 8503: ****p value<0.0001 (at 80mmHg) (Figure 2a et 2b).

### EXAMPLE 2: Parabacteroides distasonis F1-2 strain has antalgic effect in Dinitrobenzene sulfonic acid colitis mouse model:

### PARABACTEROIDES DISTASONIS CULTURING METHODS

### Cf EXAMPLE 1: PARABACTEROIDES DISTASONIS CULTURING METHODS.

### ANIMALS

25 Male C57/Blk6 mice (6-9 weeks old) were obtained from Janvier Labs (53 Le Genest-Saint-Isle. France).

### ANIMAL TREATMENTS

Colitis was induced by intrarectal instillation of Dinitrobenzene sulfonic acid (DNBS) (100mg/Kg) dissolved in 30% (v/v) ethanol solution at day 1 and day 23. During 10 days (day 17 to 26), 0.2 mL of 1.52 x 10¹⁰ CFU/mL of P. *distasonis* F1-2 strain were daily administered by gavage. Animal weight loss was followed 4 days after each DNBS intrarectal instillation to control treatment efficacy. The study groups are as follows: control (EtOH 30% + vehicle (PBS + glycerol)) group, DNBS + vehicle group (PBS + glycerol) and DNBS + F1-2 group. Colonic hypersensitivity was measured at the end of the experiment.

### MEASUREMENT COLONIC HYPERSENSITIVITY BY COLORECTAL DISTENSION

### Cf EXAMPLE 1: MEASUREMENT COLONIC HYPERSENSITIVITY BY COLORECTAL DISTENSION

### RESULTS - CONCLUSIONS

To evaluate anti-nociceptive effect of *Parabacteroides distasonis,* F1-2 strain was tested in a post-inflammatory visceral pain mouse model induced by rectal instillation of DNBS. DNBS induced a transient increase of intestinal permeability and inflammatory response during 1 to 3 days after instillation while colonic hypersensitivity was affected for a longer duration.

Beneficial effect of P. *distasonis* F1-2 was observed in post-inflammatory DNBS colitis induced mouse model (Figure 3). In fact, while DNBS rectal instillation caused colonic hypersensitivity (Figure 3a) (***p value = 0.0003 (at 80mmHg) and **p value = 0.0038 (at 60mmHg)), P. *distasonis* F1-2, administered during 10 days allowed to reverse pain phenotype (Figure 3a) (EtOH 30% + veh vs DNBS+F1-2; ns). This is also the case when we calculated AUC (Figure 3b).

### EXAMPLE 3: P. distasonis F1-2 strain has antalgic effect in IBS post-infectious visceral pain mouse model:

### PARABACTEROIDES DISTASONIS CULTURING METHODS

### Cf EXAMPLE 1: PARABACTEROIDES DISTASONIS CULTURING METHODS.

### ANIMALS

24 Male C57/Blk6 mice (6-9 weeks old) were obtained from Janvier Labs (53 Le Genest-Saint-Isle. France).

### ANIMAL TREATMENTS

After static overnight growth in a flask containing Luria broth, *Citrobacter rodentium* (ATCC 51459^{™} DBS100) cells were harvested by centrifugation and suspended in PBS buffer to reach standardized load cells of 5 x 10⁹ CFU/mL. At day 0, each mouse has been infected with 1 x 10⁹ CFU of C. *rodentium.* Infectious phase occurred before being totally clearing at day 16. Clearance of the pathogen was confirmed by counting *C. rodentium* in feces on MacConkey agar. P. *distasonis* F1-2 strain was daily-administered (3.03 x 10⁹ CFU/mice) by gavage at day 16 until day 23. The study groups are as follows: control (uninfected + vehicle (PBS + glycerol)) group (n=8), Infected mice + vehicle (PBS + glycerol) (n=9) and Infected mice + P. *distasonis* F1-2 strain (n=7). Colonic hypersensitivity was measured at the end of the experiment.

### MEASUREMENT COLONIC HYPERSENSITIVITY BY COLORECTAL DISTENSION

### Cf EXAMPLE 1: MEASUREMENT COLONIC HYPERSENSITIVITY BY COLORECTAL DISTENSION

### RESULTS - CONCLUSIONS

As colonic hypersensitivity can occur after gastro-intestinal infectious episode, antalgic potential of P. *distasonis* F1-2 strain was tested in post-infectious IBS mouse model induced by infection by the murine pathogen *Citrobacter rodentium.* 16 days post-infection, pathogen was totally cleared (data not shown) and mouse were daily gavaged with P. *distasonis* F1-2 bacterial strain during 8 days. In this model, mouse developed pain phenotype in post-infectious phase even though pathogen was totally cleared. In fact, significant colonic hypersensitivity was noted in infected group (Figure 4a) (**p value=0.0028 (at 80mmHg) and *p value=0.0202 (at 60mmHg)) (Figure 4b) (*p value=0.0267). Significant antalgic effect exerted by P. *distasonis* F1-2 was observed. In fact, colonic hypersensitivity developed by infected mouse was totally reversed by P. *distasonis* F1-2 treatment (Figure 4a) (##p value=0.002 (at 80mmHg)) (Figure 4b) (Uninfected + veh vs Infected + F1-2: ns).

### EXAMPLE 4: P. distasonis F1-2 decreases nociceptors activation level

### PARABACTEROIDES DISTASONIS CULTURING METHODS

### Cf EXAMPLE 1: PARABACTEROIDES DISTASONIS CULTURING METHODS.

### CULTURE OF PRIMARY NEURONAL DRG CELLS

Dorsal root ganglia from C57/Blk6 mouse (male) were explanted, cleaned of anterior and posterior roots and connective tissue and digested by enzyme mix containing Collagenase type III (5mg/mL, Worthington) and Dispase (10mg/mL; Gibco), for 45 minutes at 37°C. DRG suspension was centrifuged at 175 g for 15 seconds after which the supernatant was removed and replaced by 1 mL of Dulbecco's Modified Eagle Medium (DMEM; SIGMA). The DRG suspension was triturated through fire polished Pasteur pipettes and spined at 175 g 5 seconds. The supernatant containing dissociated cells was saved at each trituration step. Trituration was repeated 6 times using 3 fire polish Pasteur pipettes of decreasing diameter. Cell solution was spined for 5 min at 175 g. Supernatant was removed and replaced by 600µL of DMEM supplemented with 10% (v/v) fetal bovine serum, sodium pyruvate (1mM; Gibco), L-glutamine (2mM), and penicillin (100µg/mL), streptomycine (100µg/mL), vitamins, amino acids (MEM NEAA, Gibco), and NGF (6.25pg/mL). Cell suspension was plated on 12 poly-L-Lysine (100µg/mL) and laminin (200µg/mL) coated glass sheets in culture dishes. Cells were incubated during 30 minutes at 37°C before adding 1mL of DMEM supplemented medium in each well. Cells were incubated at 37°C overnight.

### EVALUATION OF NEURONAL CELLS ACTIVATION BY RATIOMETRIC CALCIUM IMAGING

Intracellular free Ca²⁺ was followed by ratiometric calcium imaging using Ca²⁺ ratiometric dye Fura-2 acetoxymethyl ester (Fura 2-AM, Invitrogen). DRG neurons were loaded with Fura-2 solution (4µM) supplemented by pluronic acid (1µg/mL) for 45 minutes at 37°C with slight agitation (40 rpm). All imaging experiments were performed in a dark room temperature. After loading, glass coverslip was mounted in an imaging/perfusion chamber equipped with perfusion valve system which was mounted and viewed through an inverted microscope. Neuronal cells were alternatively illuminated with 340 nm and 380 nm wavelengths. The exposure time to excitation was 400 ms for each wavelength. Image pairs were acquired every 2 seconds. Calcium imaging experiment consisted on 2 stimulations spaced by 5 min incubation with 1.99 to 2.4 x 10⁸ *P. distasonis* F1-2 viable cells/mL after being rinsed with tyrode solution. Tyrode solution was composed as followed: NaCl (140mM), KCI (3mM), MgCl₂ (1mM), CaCl₂ (2mM), D-Glucose (10mM), HEPES (10mM) adjusted at pH7.4. Osmolarity was also adjusted at 300mOsm. A lastly KCI (50mM) stimulation was assessed at the end of experiment to evaluate cell viability. KCI solution was composed as followed: NaCl (93mM), KCI (50mM), MgCl₂ (1mM), CaCl₂ (2mM), D-Glucose (10mM), HEPES (10mM) adjusted at pH7.4. Osmolarity was also adjusted at 300mOsm. In this experiment, capsaicin and P. *distasonis* F1-2 strain were diluted on tyrode physiological solution before used.

### RESULTS - CONCLUSIONS

To understand molecular mechanisms taking place between P. *distasonis* F1-2 and nociceptors, neurons from DRG mice were cultivated and loaded with Fura-2 to follow neuronal activation under nociceptive stimulations. In these experiments, primary neuronal cells from DRG mice were stimulated a first time with stimulatory solution before being incubated with vehicle or P. *distasonis* F1-2 bacterial strain for 5 minutes. After that, neuronal cells were stimulated a second time with stimulatory solution mixed with vehicle or with P. *distasonis* F1-2 bacterial strain. A lastly KCI (50mM) stimulation was made to control cell viability at the end of experiment (Figure 5a).

In the first experiment, stimulatory agent consisted on capsaicin solution targeting specifically neuronal cells expressing TRPV1 channel, implied in visceral pain. Ca²⁺ responses (measured as normalized fluorescence 340nm/380nm ratios) of each neuronal cells was followed by ratiometric calcium imaging. Quantification of the average max ΔF/F0 of capsaicin-positive nociceptive neurons revealed 2 respective groups in each condition. In fact, in each condition tested, 2 sub populations within TRPV1 positive neurons were identified. First one corresponds to TRPV1 positive neurons that responded both to first and second stimulation; represented in black (Figure 5b) whereas the second sub population corresponds to TRPV1 neurons that responded to the first stimulation only, represented in grey (Figure 5b). Neuronal cells' incubation with P. *distasonis* F1-2 significantly increased the percentage of the second sub population by turning off 26.34% of TRPV1 cells versus 4.5% for control (Figure 5b) (****p value<0,0001). This result was also reflected by a slightly calcium signal decreased in global kinetic (Figure 5c).

In the second experiment, stimulatory agent consisted on inflammatory mixture solution comprising inflammatory mediators involved in visceral pain as bradykinin, histamine, serotonin and prostaglandin 2. Results indicated that neuronal cells' incubation with P. *distasonis* F1-2 significantly increased the percentage of neuronal cells inactivated at the second stimulation applied by turning off 29,77% versus 17,39% for control (Figure 5d) (****p value<0,0001), resulting in calcium signal decreased in global kinetic (Figure 5e).

### EXAMPLE 5: P. distasonis F1-2 neuroinhibitory potential involve G-protein coupled receptor.

### PARABACTEROIDES DISTASONIS CULTURING METHODS

### Cf EXAMPLE 1: PARABACTEROIDES DISTASONIS CULTURING METHODS.

### ND7/23 IMMORTALIZED NEURONAL CELLS CULTURE AND TREATMENT

ND7/23 cells were generated by fusion of cultured neonatal rat DRG neurons with N18TG2 mouse neuroblastoma cells [19]. This immortalized cell line has been characterized as a sensory neuron model [20, 21]. ND7/23 cells were cultivated in 15 ml of Dulbecco's Modified Eagle Medium (DMEM; SIGMA) supplemented with 10% (v/v) fetal bovine serum, sodium pyruvate (110mg/mL), L-glutamine (2mM), and penicillin (100µg/mL), streptomycin (100µg/mL) in 75-cm2 culture flask. At 80% confluence, cells were detached from the substrate, centrifuged at 175 g for 5 minutes and suspended in ND7/23 fresh culture medium at 800 000 cells/mL. ND7/23 suspension was plating on 96 microplate wells (Greiner bio-one 655096) at 80 000 cells/well (100µL/well) and incubated at 37°C overnight. Experiments on ND7/23 cell line were conducted over a period of several months and spanned on average a minimum of 30-40 passages. Number of cell passages did not significantly affect responses to bradykinin stimulation.
To test involvement of Gai in direct interaction between P. *distasonis* F1-2 strain and neuronal cells, ND7/23 cells were cultivated with or without pertussis toxin (PTX, 250ng/mL, SIGMA) and incubated at 37°C overnight before used.

### NEUROMODULATORY PROPERTIES OF F1-2 ON ND7/23 IMMORTALIZED NEURONAL CELLS

After 24h of incubation, ND7/23 cells were confluent. Culture medium was removed and replaced by 81µL of calcium probe (Kit FLIPR Calcium 6-QF, Molecular Devices, λ485nm (excitation) and λ525nm (emission) and 81µL of HBSS (Hank's Buffered Salt solution)-HEPES buffer 20mM (adjusted at pH 7.4, 300mOsm). Cells were incubated with calcium probe during 2h at 37°C, following supplier recommendations. After incubation, calcium probe was removed and replaced by HBSS-HEPES buffer containing or not Ca²⁺.

To evaluate F1-2 effect on bradykinin EC50, ND7/23 cells were incubated with 1 x 10⁸ *P*. *distasonis* F1-2 viable cells/mL or vehicle (PBS + glycerol) during 45 minutes before being stimulated with bradykinin (at various final concentration). Data analysis: Maximum signal to stimulation in the second part of experiment was determined on F-F0 normalized kinetic signal. Maximum activation signal to bradykinin relative to control was represented for each bradykinin concentration tested.

### RESULTS - CONCLUSIONS

In a first experiment, immortalized ND7/23 sensory neuronal cells were incubated with P. *distasonis* F1-2 or vehicle during 45 min before being stimulated with various bradykinin concentrations (10pM to 100µM). Maximum peaks calcium signal represented on dose-response curve indicated that P. *distasonis* F1-2 reduced activation level of ND7/23 neuronal cells for all bradykinin concentration tested (Figure 6a). These results indicated P. *distasonis* F1-2 was able to interact with bradykinin receptor by decreasing activation signal to stimulation (Figure 6a). P. *distasonis* F1-2 neuro-inhibitory effect seems to be higher than those of P. *distasonis* ATCC 8503 reference strain (Figure 6a).

Neuronal activation is the result of neuronal depolarization induced in part by intracellular release of calcium whose provide by endoplasmic reticulum (RE) but also by an inlet of calcium from the extracellular compartment. In fact, in our experiment, bradykinin stimulation leads to cellular pathway activation involving GPCR, leading to an increase of intracellular calcium concentration from RE. Immortalized ND7/23 sensory neuronal cells were incubated with P. *distasonis* F1-2 bacterial strain during 45 minutes before being stimulated with bradykinin (40nM), with or without extracellular calcium (Figure 6b). Deprivation of extracellular calcium allowed to measure neuronal activation specifically linked to intracellular calcium flux. Results indicated that *P. distasonis* F1-2 strain decreased calcium signal on ND7/23 neuronal cells (Figure 6c and Figure 6d). Strongest inhibitory effect was observed when neuronal cells were deprived of extracellular calcium (Vehicle (+) Ca²⁺ vs F1-2 (+) Ca²⁺: ***p value = 0.0003. Vehicle (-) Ca²⁺ vs F1-2 (-) Ca²⁺: ****p<0.0001) (Figure 6e) suggesting that G-coupled protein receptor could be involved in bacteria-neurons interaction.

Involvement of Gai coupled protein receptor activation in neuro-inhibitory effect of P. *distasonis* F1-2 was evaluated by treating ND7/23 sensory neuronal cells with pertussis toxin (PTX). After 45 min of incubation with P. *distasonis* F1-2 strain, neuronal cells were stimulated with bradykinin (40nM). Even if Gai was blocked by PTX, inhibitory effect of P. *distasonis* F1-2 was observed (Figure 6f) with or without extracellular calcium indicating that neuro-inhibitory effect of the strain was not the result of Gai activation.

### EXAMPLE 6: P. distasonis F1-2 neuroinhibitory potential could involve bradykinin receptor

### PARABACTEROIDES DISTASONIS CULTURING METHODS

### Cf EXAMPLE 1: PARABACTEROIDES DISTASONIS CULTURING METHODS.

### CULTURE OF PRIMARY NEURONAL DRG CELLS

### Cf EXAMPLE 4: CULTURE OF PRIMARY NEURONAL DRG CELLS

### EVALUATION OF NEURONAL CELLS ACTIVATION BY RATIOMETRIC CALCIUM IMAGING

### Cf EXAMPLE 4: EVALUATION OF NEURONAL CELLS ACTIVATION BY RATIOMETRIC CALCIUM IMAGING

### RESULTS - CONCLUSIONS

Contribution of bradykinin receptors in P. *distasonis* F1-2 neuroinhibitory potential was evaluated on primary neuronal cells from DRG mice. Primary neuronal cells were stimulated a first time with stimulatory solution before being incubated during 5 minutes with vehicle or with P. *distasonis* F1-2. Then, neuronal cells were stimulated a second time with stimulatory agent mixed with vehicle or P. *distasonis* F1-2 strain. A lastly KCI (50mM) stimulation was made to control cell viability at the end of experiment (Figure 7a). In the first experiment, stimulatory agent consisted on inflammatory soap (IS) solution targeting G-coupled protein receptors comprising bradykinin (IS (+) Brady) or not (IS (-) Brady), or bradykinin alone. Ca²⁺ responses (measured as normalized fluorescence 340nm/380nm ratios) of each neuronal cell was followed by ratiometric calcium imaging. Quantification of the average max ΔF/F0 of IS-positive nociceptive neurons revealed 2 respective groups in each condition. In fact, in each condition tested, 2 sub populations within responsive neurons to stimulation applied were identified. First one corresponds to responsive neurons that responded both to first and second stimulation; represented in black (Figure 7b) whereas the second sub population corresponds to neurons that responded only to the first stimulation, represented in grey (Figure 7b). Neuro-inhibitory potential of P. *distasonis* F1-2, reflected by significant increase of neuronal cells inactivated at the second stimulation, was observed only when stimulation contained bradykinin. In fact, when neuronal cells were stimulated with IS containing bradykinin, *P. distasonis* F1-2 cell's incubation leaded to increase inactivated neuronal cells, turning off 29,96% of responsive cells versus 13,62% for control (Figure 7b) (****p value<0,0001). Surprisingly, inhibitory effect was lost when IS was deprived of bradykinin (Figure 7b) while under bradykinin stimulation, neuro-inhibitory effect was observed, resulting in an increased of inactivated neuronal cells turning off 82,93% versus 39,45% for control (Figure 7b) (****p value<0,0001).

Bradykinin receptor is a GPCR that recruiting Gai or Gαq protein. While neuronal inhibition requires α activation in case of Gai protein coupled receptor, inhibition of a subunit is required in case of Gαq protein coupled receptor. To evaluate interaction specificity between *P. distasonis* F1-2 strain and bradykinin receptor, the same experiment was conducted by replacing bradykinin stimulation by specific agonist of histamine receptor 1 or H1R (2-pyridilethylamine), which is a Gαq coupled protein receptor as bradykinin receptor. Although P. *distasonis* F1-2 significantly increased the percentage of no responding cells to the second bradykinin stimulation (Figure 7c) (****p value<0,0001), neuroinhibitory effect was not observed with 2-pyridylethylamine stimulation (Figure 6c) suggesting that H1 receptor was not involved in *P. distasonis* F1-2 and neuronal cells direct interaction. In addition, this result suggested that P. *distasonis* F1-2 does not interact specifically with Gαq protein because inhibitory effect was not observed with H1R stimulation.
Name of depositary Institution: Collection nationale de cultures de micro-organismes (CNCM)
Adress of depositary Institution : Institut Pasteur, 28, rue du Dr Roux, 75724 Paris Cedex 15
Accession Number: CNCM I-5828
Date of deposit: February 16, 2022

### Explanation of sequence listing

SEQ ID NO:1 Parabacteroides distasonis strain F1-2 according to the invention, gene for 16S ribosomal RNA.

### References

1. Marchesi, J. R. & Ravel, J. The vocabulary of microbiome research: a proposal. Microbiome 3, 31 (2015).
2. Eckburg, P. B. et al. Diversity of the human intestinal microbial flora. Science 308, 1635-1638 (2005).
3. Sender, R., Fuchs, S. & Milo, R. Are We Really Vastly Outnumbered? Revisiting the Ratio of Bacterial to Host Cells in Humans. Cell 164, 337-340 (2016).
4. Sender, R., Fuchs, S. & Milo, R. Revised Estimates for the Number of Human and Bacteria Cells in the Body. PLOS Biology 14, e1002533 (2016).
5. Rinninella, E. et al. What is the Healthy Gut Microbiota Composition? A Changing Ecosystem across Age, Environment, Diet, and Diseases. Microorganisms 7, E14 (2019).
6. Casén, C. et al. Deviations in human gut microbiota: a novel diagnostic test for determining dysbiosis in patients with IBS or IBD. Aliment Pharmacol Ther 42, 71-83 (2015).
7. Li, J., Butcher, J., Mack, D. & Stintzi, A. Functional impacts of the intestinal microbiome in the pathogenesis of inflammatory bowel disease. Inflamm Bowel Dis 21, 139-153 (2015).
8. Lloyd-Price, J. et al. Multi-omics of the gut microbial ecosystem in inflammatory bowel diseases. Nature 569, 655-662 (2019).
9. Caenepeel, C., Sadat Seyed Tabib, N., Vieira-Silva, S. & Vermeire, S. Review article: how the intestinal microbiota may reflect disease activity and influence therapeutic outcome in inflammatory bowel disease. Aliment Pharmacol Ther 52, 1453-1468 (2020).
10. Aghazadeh, R. et al. Inflammatory bowel disease in Iran: a review of 457 cases. J Gastroenterol Hepatol 20, 1691-1695 (2005).
11. Ceuleers, H. et al. Visceral hypersensitivity in inflammatory bowel diseases and irritable bowel syndrome: The role of proteases. World J Gastroenterol 22, 10275-10286 (2016).
12. Jones, R. C. W., Xu, L. & Gebhart, G. F. The mechanosensitivity of mouse colon afferent fibers and their sensitization by inflammatory mediators require transient receptor potential vanilloid 1 and acid-sensing ion channel 3. J Neurosci 25, 10981-10989 (2005).
13. Akbar, A. et al. Expression of the TRPV1 receptor differs in quiescent inflammatory bowel disease with or without abdominal pain. Gut 59, 767-774 (2010).
14. Akbar, A. et al. Increased capsaicin receptor TRPV1-expressing sensory fibres in irritable bowel syndrome and their correlation with abdominal pain. Gut 57, 923-929 (2008).
15. Miranda, A. et al. The role of transient receptor potential vanilloid 1 in mechanical and chemical visceral hyperalgesia following experimental colitis. Neuroscience 148, 1021-1032 (2007).
16. Gottesman-Katz, L., Latorre, R., Vanner, S., Schmidt, B. L. & Bunnett, N. W. Targeting G protein-coupled receptors for the treatment of chronic pain in the digestive system. Gut 70, 970-981 (2021).
17. Sakamoto, M. & Benno, Y. Reclassification of Bacteroides distasonis, Bacteroides goldsteinii and Bacteroides merdae as Parabacteroides distasonis gen. nov., comb. nov., Parabacteroides goldsteinii comb. nov. and Parabacteroides merdae comb. nov. International Journal of Systematic and Evolutionary Microbiology 56, 1599-1605 (2006).
18. Hiippala, K. et al. Isolation of Anti-Inflammatory and Epithelium Reinforcing Bacteroides and Parabacteroides Spp. from A Healthy Fecal Donor. Nutrients 12, 935 (2020).
19. Wood, J. N. et al. Novel cell lines display properties of nociceptive sensory neurons. Proceedings of the Royal Society of London. Series B: Biological Sciences 241, 187-194 (1990).
20. Haberberger, R. V., Barry, C. & Matusica, D. Immortalized Dorsal Root Ganglion Neuron Cell Lines. Front. Cell. Neurosci. 14, (2020).
21. Kobrinsky, E. M., Pearson, H. A. & Dolphin, A. C. Low- and high-voltage-activated calcium channel currents and their modulation in the dorsal root ganglion cell line ND7-23. Neuroscience 58, 539-552 (1994).

## Claims

1. A *Parabacteroides distasonis* strain deposited under accession number CNCM I-5828.

2. The *Parabacteroides distasonis* strain according to claim 1, wherein the 16s rRNA gene sequence of the *Parabacteroides distasonis* strain is as shown in SEQ ID NO:1.

3. The *Parabacteroides distasonis* strain according to any one of claims 1 and 2, wherein said strain is isolated from healthy human feces.

4. A nutritional supplement composition comprising a bacterial strain according to any one of the preceding claims.

5. The nutritional supplement composition according to the preceding claim, further comprising at least one acceptable nutritional ingredient, preferably chosen among: probiotics, prebiotics, symbiotics, parabiotics, metabiotics vitamins, minerals, herbs, amino acids and enzymes.

6. A composition comprising a bacterial strain according to any one of claims 1 to 3, for use as a medicament.

7. The composition for use according to the preceding claim, for use in treating and/or preventing visceral pain.

8. The composition for use according to the preceding claim, wherein said visceral pain is induced by Inflammatory Bowel Disease including Crohn's disease and Ulcerative Colitis, or by Irritable Bowel Syndrome.

9. The nutritional supplement composition according to any one of claims 4 to 5, or the composition for use according to any one of claims 6 to 8, wherein the composition comprises at least 10⁹, preferably at least 10¹⁰, colony forming units/mL of the *Parabacteroides distasonis* strain deposited under accession number CNCM 1-5828.

10. The nutritional supplement composition according to any one of claims 4, 5 and 9, or the composition for use according to any one of claims 6 to 9, wherein the composition only comprises living bacteria.

11. The nutritional supplement composition according to any one of claims 4, 5 and 9, or the composition for use according to any one of claims 6 to 9, wherein the composition only comprises killed bacteria and/or fractions of bacteria.

12. The composition for use according to any one of claims 6 to 11, further comprising at least one other acceptable active pharmaceutical ingredient, and/or at least one acceptable pharmaceutical excipient and/or at least one acceptable pharmaceutical carrier.

13. The composition for use according to any one of claims 6 to 12, wherein the composition is for oral, or in intestine administration, preferably oral administration.

14. The composition for use according to the preceding claim, wherein the oral administration is made by gelatin capsules, capsules, tablets, powders, granules, oral solutions or suspensions. ]

## Patentansprüche

1. *Parabacteroides* distasonis-Stamm, hinterlegt unter der Zugangsnummer CNCM 1-5828.

2. *Parabacteroides* distasonis-Stamm nach Anspruch 1, wobei die 16s-rRNA-Gensequenz des *Parabacteroides distasonis-Stamms* wie in SEQ ID NO:1 gezeigt ist.

3. *Parabacteroides distasonis-Stamm* nach einem der Ansprüche 1 und 2, wobei der Stamm aus dem Stuhl gesunder Menschen isoliert wird.

4. Nahrungsergänzungszusammensetzung, umfassend einen Bakterienstamm nach einem der vorstehenden Ansprüche.

5. Nahrungsergänzungszusammensetzung nach dem vorstehenden Anspruch, ferner umfassend mindestens einen akzeptablen Nahrungsbestandteil, der vorzugsweise ausgewählt wird aus: Probiotika, Präbiotika, Symbiotika, Parabiotika, Metabiotika, Vitaminen, Mineralien, Kräutern, Aminosäuren und Enzymen.

6. Zusammensetzung, umfassend einen Bakterienstamm nach einem der Ansprüche 1 bis 3 zur Verwendung als ein Medikament.

7. Zusammensetzung zur Verwendung nach dem vorstehenden Anspruch zur Verwendung bei einem Behandeln und/oder Vorbeugen von viszeralen Schmerzen.

8. Zusammensetzung zur Verwendung nach dem vorstehenden Anspruch, wobei der viszerale Schmerz durch eine entzündliche Darmerkrankung, einschließlich Morbus Crohn und Colitis ulcerosa, oder durch ein Reizdarmsyndrom hervorgerufen wird.

9. Nahrungsergänzungszusammensetzung nach einem der Ansprüche 4 bis 5 oder Zusammensetzung zur Verwendung nach einem der Ansprüche 6 bis 8, wobei die Zusammensetzung mindestens 10⁹, vorzugsweise mindestens 10¹⁰, koloniebildende Einheiten/ml des *Parabacteroides* distasonis-Stamms, hinterlegt unter der Zugangsnummer CNCM 1-5828, umfasst.

10. Nahrungsergänzungszusammensetzung nach einem der Ansprüche 4, 5 und 9 oder Zusammensetzung zur Verwendung nach einem der Ansprüche 6 bis 9, wobei die Zusammensetzung nur lebende Bakterien umfasst.

11. Nahrungsergänzungszusammensetzung nach einem der Ansprüche 4, 5 und 9 oder Zusammensetzung zur Verwendung nach einem der Ansprüche 6 bis 9, wobei die Zusammensetzung nur abgetötete Bakterien und/oder Bakterienfraktionen umfasst.

12. Zusammensetzung zur Verwendung nach einem der Ansprüche 6 bis 11, ferner umfassend mindestens einen anderen akzeptablen pharmazeutischen Wirkstoff und/oder mindestens einen akzeptablen pharmazeutischen Hilfsstoff und/oder mindestens einen akzeptablen pharmazeutischen Träger.

13. Zusammensetzung zur Verwendung nach einem der Ansprüche 6 bis 12, wobei die Zusammensetzung für eine orale oder intestinale Verabreichung, vorzugsweise für die orale Verabreichung, bestimmt ist.

14. Zusammensetzung zur Verwendung nach dem vorstehenden Anspruch, wobei die orale Verabreichung durch Gelatinekapseln, Kapseln, Tabletten, Pulvern, Granulate, orale Lösungen oder Suspensionen erfolgt.

## Revendications

1. Souche de *Parabacteroides distasonis* déposée sous le numéro d'accession CNCM I-5828.

2. Souche de *Parabacteroides distasonis* selon la revendication 1, dans laquelle la séquence du gène ARNr 16s de la souche de *Parabacteroides distasonis* est telle que présentée dans la séquence SEQ ID NO : 1.

3. Souche de *Parabacteroides distasonis* selon l'une quelconque des revendications 1 et 2, dans laquelle ladite souche est isolée à partir de matières fécales humaines saines.

4. Composition de complément nutritionnel comprenant une souche bactérienne selon l'une quelconque des revendications précédentes.

5. Composition de complément nutritionnel selon la revendication précédente, comprenant en outre au moins un ingrédient nutritionnel acceptable, de préférence choisi parmi : probiotiques, prébiotiques, symbiotiques, parabiotiques, métabiotiques, vitamines, minéraux, herbes, acides aminés et enzymes.

6. Composition comprenant une souche bactérienne selon l'une quelconque des revendications 1 à 3, destinée à être utilisée en tant que médicament.

7. Composition destinée à être utilisée selon la revendication précédente, destinée à être utilisée dans le traitement et/ou la prévention de la douleur viscérale.

8. Composition destinée à être utilisée selon la revendication précédente, dans laquelle ladite douleur viscérale est induite par une maladie inflammatoire de l'intestin, y compris la maladie de Crohn et la colite ulcéreuse, ou par le syndrome de l'intestin irritable.

9. Composition de complément nutritionnel selon l'une quelconque des revendications 4 à 5, ou composition destinée à être utilisée selon l'une quelconque des revendications 6 à 8, dans laquelle la composition comprend au moins 10⁹, de préférence au moins 10¹⁰, unités formant colonies/ml de la souche de *Parabacteroides distasonis* déposée sous le numéro d'accession CNCM I-5828.

10. Composition de complément nutritionnel selon l'une quelconque des revendications 4, 5 et 9, ou composition destinée à être utilisée selon l'une quelconque des revendications 6 à 9, dans laquelle la composition ne comprend que des bactéries vivantes.

11. Composition de complément nutritionnel selon l'une quelconque des revendications 4, 5 et 9, ou composition destinée à être utilisée selon l'une quelconque des revendications 6 à 9, dans laquelle la composition ne comprend que des bactéries tuées et/ou des fractions de bactéries.

12. Composition destinée à être utilisée selon l'une quelconque des revendications 6 à 11, comprenant en outre au moins un autre ingrédient pharmaceutique actif acceptable, et/ou au moins un excipient pharmaceutique acceptable et/ou au moins un support pharmaceutique acceptable.

13. Composition destinée à être utilisée selon l'une quelconque des revendications 6 à 12, dans laquelle la composition est destinée à être administrée par voie orale ou dans l'intestin, de préférence par voie orale.

14. Composition destinée à être utilisée selon la revendication précédente, dans laquelle l'administration par voie orale se fait avec des gélules de gélatine, des gélules, des comprimés, des poudres, des granulés, des solutions orales ou des suspensions.
